(19) Europäisches Patentamt
European Patent Office
Office européen des brevets

(11) Veröffentlichungsnummer: **0 487 986 B1**

(12) # EUROPÄISCHE PATENTSCHRIFT

(45) Veröffentlichungstag der Patentschrift: **21.06.95**

(21) Anmeldenummer: **91119430.6**

(22) Anmeldetag: **14.11.91**

(51) Int. Cl.[6]: **C07C 69/675**, C07C 67/31, C12P 7/62, C07C 69/716, C07D 339/04

(54) **Verfahren zur Herstellung von (6s)-6,8-Dihydroxyoctansäureestern.**

(30) Priorität: **24.11.90 DE 4037440**

(43) Veröffentlichungstag der Anmeldung:
**03.06.92 Patentblatt 92/23**

(45) Bekanntmachung des Hinweises auf die Patenterteilung:
**21.06.95 Patentblatt 95/25**

(84) Benannte Vertragsstaaten:
**CH DE ES FR GB IT LI SE**

(56) Entgegenhaltungen:

**Keine einschl gigen Dokumente gefunden**

(73) Patentinhaber: **BASF Aktiengesellschaft**
**Carl-Bosch-Strasse 38**
**D-67063 Ludwigshafen (DE)**

(72) Erfinder: **Balkenhohl, Friedhelm, Dr.**
**Trifelsring 37**
**W-6703 Limburgerhof (DE)**
Erfinder: **Paust, Joachim, Dr.**
**Ringstrasse 3**
**W-6708 Neuhofen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 487 986 B1

**Beschreibung**

Die vorliegende Erfindung betrifft ein neues Verfahren zur Herstellung von (6S)-6,8-Dihydroxyoctansäureestern der allgemeinen Formel I

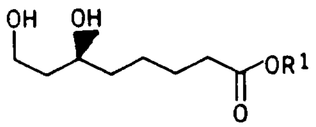

I

in der
$R^1$ eine Alkyl-, Cycloalkyl-, Aralkyl- oder Arylgruppe bezeichnet.

Außerdem betrifft die Erfindung ein Verfahren zur Herstellung von (3S)-3-Hydroxyoctandisäurediestern.

Des weiteren betrifft die Erfindung neue (3S)-3-Hydroxyoctandisäurediester und neue 3-Oxooctandisäurediester sowie ein Verfahren zur Herstellung von R-(+)-α-Liponsäure.

Die Verbindungen I sind bekannt und dienen als Zwischenprodukte für die Synthese von enantiomerenreiner R-(+)-α-Liponsäure (Thioctsäure).

In J. Chem. Soc. Perkin Trans., I, 9 (1988) ist ihre Herstellung aus dem Äpfelsäurederivat (S)-4-phenylmethoxybutan-1,2-diol über eine mehrstufige Reaktionsfolge mit den Zwischenprodukten (R)-4-phenyl-methoxybutan-1,2-diol, (R)-(2-phenylmethoxyethyl)oxiran, 6-Hydroxy-8-(phenylmethoxy)-oct-1-en zum (6S)-6,8-Dihydroxyoctansäuremethylester beschrieben.

Aus Tetrahedron Letters₁ 42, 5705 (1989) ist die Alkylierung des Acetessigesterbisanions zum 3-Oxo-7-cyano-heptansäureester, dessen Reduktion mit Bäckerhefe zum (3S)-3-Hydroxy-7-cyano-heptansäureester, die nachfolgende Reduktion dieser Verbindung mit Lithiumborhydrid in Tetrahydrofuran sowie Alkoholyse zum (6S)-6,8-Dihydroxyoctansäureethylester bekannt. In dieser Literaturstelle ist auch die Herstellung der R-(+)-α-Liponsäure beschrieben. Eine weitere Synthesemöglichkeit der R-(+)-α-Liponsäure ist die mehrstufige Umsetzung von Propargylalkohol über die Zwischenprodukte E-Nona-2,8-dien-1-ol, 2S,3S-2-Epoxy-1-hydroxynon-8-en, 1,3-Dihydroxynon-8-en, 1,3-Disulfoxynon-8-en und 6,8-Disulfoxyoctansäure (DE-A 36 29 116).

Diese bisher bekannten Verfahren vermögen jedoch trotz hoher chemischer Ausbeuten sowohl aufgrund des hohen technischen Aufwandes als auch ungenügender optischer Reinheit nicht zu befriedigen.

Der Erfindung lag deshalb die Aufgabe zugrunde, die (6S)-6,8-Dihydroxyoctansäureester I auf einfachere und wirtschaftlichere Weise in hoher optischer und chemischer Ausbeute zugänglich zu machen sowie weitere Ausgangsstoffe hierfür zur Verfügung zu stellen.

Demgemäß wurde ein neues Verfahren zur Herstellung von (6S)-6,8-Dihydroxyoctansäureestern der allgemeinen Formel I

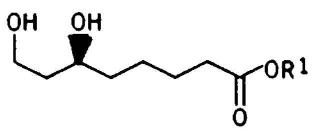

I

in der
$R^1$ eine Alkyl-, Cycloalkyl-, Aralkyl- oder Arylgruppe bezeichnet,
gefunden, welches dadurch gekennzeichnet ist, daß man einen (3S)-3-Hydroxyoctandisäurediester der allgemeinen Formel II

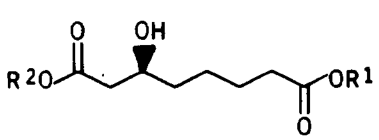

II

in der $R^2$ einen der Reste $R^1$ bedeutet, mit einem komplexen Hydrid reduziert.

Weiterhin wurde ein Verfahren zur Herstellung von (3S)-3-Hydroxyoctandisäurediestern, neue (3S)-3-Hydroxyoctandisäurediester und neue 3-Oxooctandisäurediester sowie ein Verfahren zur Herstellung von R-(+)-α-Liponsäure gefunden.

Die erfindungsgemäß einzusetzenden (3S)-3-Hydroxyoctandisäurediester II sind neu. Unter diesen Verbindungen werden solche der Formel IIa bevorzugt, in denen die Reste $R^{1a}$ und $R^{2a}$ die folgende Bedeutung haben:

- $C_1$-$C_{20}$-Alkylgruppen, vorzugsweise $C_1$-$C_8$-Alkylgruppen, darunter vorzugsweise $C_1$-$C_5$-Alkylgruppen wie Ethyl, n-Propyl, iso-Propyl, n-Butyl, n-Pentyl und besonders Methyl und iso-Butyl;
- $C_3$-$C_{12}$-Cycloalkylgruppen, vorzugsweise $C_3$-$C_8$-Cycloalkylgruppen wie Cyclopropyl, Cyclobutyl, Cyclopentyl, Cycloheptyl, Cyclooctyl und besonders Cyclohexyl;
- $C_7$-$C_{12}$-Aralkylgruppen, vorzugsweise Phenylethyl und ganz besonders Benzyl;
- ein- oder zweikernige Arylgruppen wie Naphthyl und besonders Phenyl.

Hierbei können die Alkyl-, Cycloalkyl-, Aralkyl- und Arylgruppen ihrerseits vorzugsweise bis zu 2 Substituenten tragen, insbesondere $C_1$-$C_{12}$-Alkylgruppen, $C_1$-$C_{12}$-Alkoxygruppen und Halogen.

Bevorzugte Ausgangsstoffe II sind:

-(3S)-3-Hydroxyoctandisäuredimethylester
-(3S)-1-Ethyl-3-hydroxy-8-methyl-octandioat
-(3S)-3-Hydroxy-8-methyl-1-propyl-octandioat
-(3S)-3-Hydroxy-8-methyl-1-iso-propyl-octandioat
-(3S)-1-Butyl-3-hydroxy-8-methyl-octandioat
-(3S)-3-Hydroxy-1-sec.-butyl-8-methyl-octandioat
-(3S)-3-Hydroxy-8-methyl-1-tert.-butyl-octandioat
-(3S)-3-Hydroxy-8-methyl-1-octyl-octandioat
-(3S)-3-Hydroxy-8-methyl-1-phenyl-octandioat
-(3S)-1-(2-Ethylhexyl)-3-hydroxy-8-methyl-octandioat

Im Hinblick auf die gewünschten Verfahrensprodukte I ist das (3S)-3-Hydroxy-1-iso-butyl-8-methyl-octandioat besonders bevorzugt.

Die Reduktion der Ausgangsverbindungen II zu den Verfahrensprodukten I erfolgt mit einem komplexen Hydrid, wobei das Molverhältnis des komplexen Hydrides zu II zweckmäßigerweise 1:1 bis 3:1, besonders 1,5:1 bis 2,5:1 beträgt.

Als bevorzugte komplexe Hydride sind Borhydride wie Lithium- und Kaliumborhydrid und besonders bevorzugt Natriumborhydrid zu nennen.

Weiterhin eignen sich vor allem alkyl- und alkoxysubstituierte Borhydride wie Lithiumtriethylborhydrid und Natriumtrimethoxyborhydrid.

Es empfiehlt sich, die Reduktion von II zu I in einem aprotischen Lösungsmittel vorzunehmen. Als solche eignen sich bevorzugt aliphatische und aromatische Kohlenwasserstoffe wie Hexan, Cyclohexan, Toluol, Benzol und Xylol und Ether wie Dioxan, Diethylether und besonders bevorzugt Tetrahydrofuran.

Im allgemeinen führt man die Reaktion bei 0 bis 150 °C, vorzugsweise bei 25 bis 70 °C durch.

Die Umsetzung wird in der Regel bei Normaldruck vorgenommen, jedoch kann man auch bei vermindertem oder leicht erhöhtem Druck arbeiten, also etwa im Bereich von 0,1 bis 10 bar.

Die Reaktionszeiten betragen normalerweise 0,5 bis 5 Stunden, meistens 1 bis 2 Stunden.

Verfahrenstechnisch geht man im allgemeinen so vor, daß man eine Lösung der Ausgangsverbindung II in dem aprotischen Lösungsmittel mit dem komplexen Hydrid versetzt und die entstehende Suspension erhitzt.

Man kann die Aufarbeitung des Reaktionsgemisches auf die Verfahrensprodukte I in an sich bekannter Weise vornehmen, und zwar in der Regel durch Hydrolyse, Extraktion und Trocknung.

Die Ausgangsverbindungen II können erfindungsgemäß aus den 3-Oxooctandisäureestern III hergestellt werden.

Die Verbindungen III sind zum Teil bekannt oder nach bekannten Methoden erhältlich, darunter vor allem durch Acylierung von Meldrumsäure mit Adipinsäuremonoalkylesterchlorid und anschließende Alkoholyse (für $R^1$ und $R^2$ = Methyl: Liebigs Ann. Chem. 1237 (1983); für $R^1$ = Methyl und $R^2$ = tert.-Butyl: Chem. Ber., 122, 797 (1989); für $R^1$ und $R^2$ = Ethyl: Org. Prep. Proced. Int., 20, 184 (1988).

Ganz besondere Bedeutung ist den neuen Verbindungen IIIb beizumessen, in denen die Reste $R^{1b}$ und $R^{2b}$ folgende Bedeutung haben:

- $C_1$-$C_{20}$-Alkylgruppen, ausgenommen die Methylgruppe für $R^{1b}$ und die Methyl- oder tert.-Butylgruppe für $R^{2b}$ sowie die Ethylgruppe für $R^{1b}$ und $R^{2b}$, vorzugsweise $C_1$-$C_8$-Alkylgruppen, darunter vorzugsweise $C_1$-$C_5$-Alkylgruppen wie Ethyl, n-Propyl, iso-Propyl, n-Butyl, n-Pentyl und besonders Methyl und iso-Butyl;
- $C_3$-$C_{12}$-Cycloalkylgruppen, vorzugsweise $C_3$-$C_8$-Cycloalkylgruppen wie Cyclopropyl, Cyclobutyl, Cyclopentyl, Cycloheptyl, Cyclooctyl und besonders Cyclohexyl;

- $C_7$-$C_{12}$-Aralkylgruppen, vorzugsweise Phenylethyl und ganz besonders Benzyl;
- ein- oder zweikernige Arylgruppen wie Naphthyl und besonders Phenyl.

Hierbei können die Alkyl-, Cycloalkyl-, Aralkyl- und Arylgruppen ihrerseits vorzugsweise bis zu 2 Substituenten tragen, insbesondere $C_1$-$C_{12}$-Alkylgruppen, $C_1$-$C_{12}$-Alkoxygruppen und Halogen.

Bevorzugte Ausgangsstoffe IIIb sind:

-1-Ethyl-8-methyl-3-oxo-octandioat
-8-Methyl-3-oxo-1-propyl-octandioat
-1-Iso-propyl-8-methyl-3-oxo-octandioat
-1-Butyl-8-methyl-3-oxo-octandioat
-8-Methyl-3-oxo-1-sec.-butyl-octandioat
-8-Methyl-1-octyl-3-oxo-octandioat
-8-Methyl-3-oxo-1-phenyl-octandioat
-1-(2-Ethylhexyl)-8-methyl-3-oxo-octandioat

Im Hinblick auf die gewünschten Verfahrensprodukte I und II ist das 1-Iso-butyl-8-methyl-3-oxo-octandioat besonders bevorzugt.

Die Reduktion der Verbindungen III zu den Verbindungen II erfolgt mit Bäckerhefe, die man vorzugsweise in wasserfreier Form als Trockenhefe einsetzt. Das Mengenverhältnis von Bäckerhefe zu III beträgt zweckmäßigerweise 1:1 bis 100:1, bevorzugt 4:1 bis 20:1.

Zweckmäßigerweise führt man die Reduktion in wäßriger Lösung durch.

Es empfiehlt sich, Alkohole wie vorzugsweise Ethanol oder Zucker als Energielieferanten zur Aufrechterhaltung der Reduktion zuzusetzen.

In der Regel führt man die Bäckerhefereduktion bei 0 bis 50 °C, vorzugsweise bei 30 bis 40 °C durch.

Zweckmäßigerweise arbeitet man bei Normaldruck.

Die Reaktionszeiten betragen im allgemeinen 12 bis 96 Stunden, meistens 24 bis 48 Stunden.

Verfahrenstechnisch geht man üblicherweise so vor, daß man die Bäckerhefe in der wäßrig-alkoholischen Lösung suspendiert und nach dem Erwärmen mit der Verbindung III versetzt.

Die Aufarbeitung des Reaktionsgemisches auf die Verfahrensprodukte II nimmt man wie üblich vor, und zwar in der Regel durch Filtration, Extraktion des Filtrates und anschließende Trocknung der Extraktphase.

Die Verbindungen II dienen zur Herstellung von R-(+)-α-Liponsäure der Formel IV

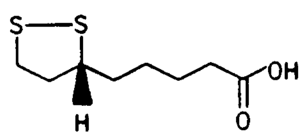

IV

indem sie zu den Verbindungen I reduziert werden und diese

a) in organischer Lösung mit einem Sulfonsäurechlorid und einer tertiären Stickstoffbase in den Bissulfonsäureester von I überführt werden,
b) diese Verbindung in einem polaren Lösungsmittel mit Schwefel und einem Alkalimetalldisulfid zum R-α-Liponsäureester umgesetzt wird und
c) dieser Ester gewünschtenfalls in die physiologisch verträgliche R-(+)-α-Liponsäure überführt wird.

Das Verfahrensprodukt IV ist bekannt oder nach bekannten Methoden erhältlich, darunter vor allem durch die bereits beschriebene Reaktionsfolge vom Acetessigesterbisanion zum (6S)-6,8-Dihydroxyoctansäureester sowie dessen anschließende Umsetzung über die Stufen a, b und c (Tetrahedron Letters, 42, 5705 (1989)).

Die erfindungsgemäßen Verbindungen II und die nach dem erfindungsgemäßen Verfahren hergestellte Verbindung I weisen in der Regel ein hohes Enantiomerenverhältnis auf, entsprechend einer optischen Ausbeute von 20 bis 94 %.

Die Enantiomerenverhältnisse werden nach Dale, Dull und Mosher, J. Org. Chem., 34, 2543 (1969) durch Gaschromatographie der diastereomeren Ester aus den erfindungsgemäßen Alkoholen und einer optisch aktiven α-Methoxy-α-phenyl-α-trifluoromethylessigsäure gemessen.

Die nach dem Verfahren der Erfindung hergestellten (6S)-6,8-Dihydroxyoctansäureester I finden hauptsächlich als Zwischenprodukte für die Synthese von enantiomerenreiner R-(+)-α-Liponsäure Verwendung. Diese wird als Racemat im wesentlichen zur Behandlung akuter und chronischer Lebererkrankungen sowie bei Vergiftungen eingesetzt. Da ausschließlich das natürliche R-(+)-α-Enantiomere die biologische Aktivität aufweist, ist die asymmetrische Synthese dieses reinen Naturstoffes von großer Wichtigkeit.

Das erfindungsgemäße Verfahren zur Herstellung der Verbindungen I zeichnet sich gegenüber dem Stand der Technik durch eine verfahrenstechnisch einfache Durchführung, gute Ausbeuten und hohe Produktreinheit aus, da mit den Verbindungen IIa neue Ausgangsstoffe für die Synthese der Verbindungen I zur Verfügung stehen.

Die vorliegende Erfindung ermöglicht es, die physiologisch wichtige R-(+)-α-Liponsäure auf einfache und wirtschaftliche Weise in hoher optischer Ausbeute zugänglich zu machen.

Die Verbindungen II werden erfindungsgemäß aus den Verbindungen III hergestellt, wobei insbesondere die neuen Verbindungen IIIb hohe optische Ausbeuten der Verbindungen II bewirken.

Beispiele

Beispiele 1 bis 6

A) Herstellung von (3S)-3-Hydroxyoctandisäurediestern der Formel

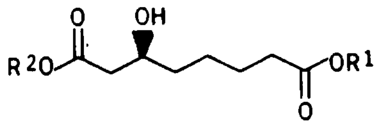

II

Eine Lösung aus 20 ml Ethanol (abs.) in 1000 ml Wasser wurde mit 42 g Trocken-Bäckerhefe versetzt und innerhalb von 0,5 Stunden auf 35 °C erwärmt. Danach wurden 10 g der Verbindung III zugesetzt und die Lösung für weitere 24 bis 72 Stunden bei 36 °C gerührt.

Die Hefe wurde abgetrennt, zunächst mit 150 ml Wasser und dann mit 450 ml Hexan gewaschen. Nach der Extraktion mit 800 ml Hexan wurde der Extrakt mit Natriumhydrogencarbonat nachgewaschen und schließlich getrocknet.

Die Einzelheiten dieser Versuche sowie deren Ergebnisse sind der nachstehenden Tabelle zu entnehmen.

Beispiel 7

B) Herstellung von (6S)-6,8-Dihydroxyoctansäuremethylester

Eine Lösung aus 50 mmol (3S)-3-Hydroxy-1-isobutyl-8-methyl-octandioat in 100 ml Tetrahydrofuran wurde mit 100 mmol Natriumborhydrid versetzt, und die entstehende Suspension wurde für die Dauer von 2 Stunden auf 65 °C erhitzt.

Nach Abkühlung auf Raumtemperatur wurde die Lösung mit 200 ml gesättigter Natriumhydrogencarbonatlösung neutralisiert, dann mit 400 ml Essigester extrahiert und schließlich getrocknet. Der Rückstand wurde erneut in 150 ml Methanol gelöst, wonach die Lösung für die Dauer von 2 Stunden auf 64 °C erhitzt wurde. Nach Entfernung des Methanols wurde abschließend getrocknet.

Man erhielt das Produkt I in einer Ausbeute von 88 Gew.-% und in einem Enantiomerenverhältnis von 97:3.

Tabelle

Herstellung von (3S)-3-Hydroxyoctandisäurediestern II

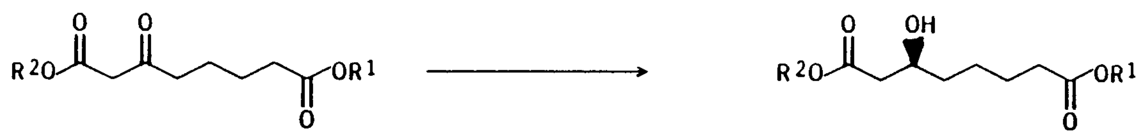

III II

| Beispiel | $R^1$ | $R^2$ | Ausbeute an II [Gew.-%] | Enantiomerenverhältnis |
|---|---|---|---|---|
| 1 | Methyl | Methyl | 45 | 85:15 |
| 2 | Methyl | iso-Propyl | 70 | 60:40 |
| 3 | Methyl | n-Butyl | 65 | 65:35 |
| 4 | Methyl | iso-Butyl | 70 | 97:3 |
| 5 | Methyl | sec.-Butyl | 60 | 65:35 |
| 6 | Methyl | iso-Pentyl | 55 | 70:30 |

## Patentansprüche

**Patentansprüche für folgende Vertragsstaaten : CH, DE, FR, GB, IT, LI, SE**

1. Verfahren zur Herstellung von (6S)-6,8-Dihydroxyoctansäureestern der allgemeinen Formel I

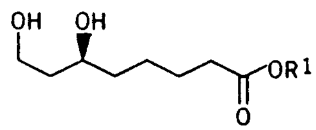

I

in der
$R^1$ eine Alkyl-, Cycloalkyl-, Aralkyl- oder Arylgruppe bezeichnet, dadurch gekennzeichnet, daß man einen (3S)-3-Hydroxyoctandisäurediester der allgemeinen Formel II

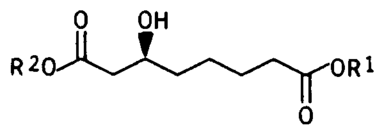

II

in der $R^2$ einen der Reste $R^1$ bedeutet, mit einem komplexen Hydrid reduziert.

2. Verfahren nach Anspruch 1, dadurch gekennzeichnet, daß man als komplexes Hydrid Natriumborhydrid verwendet.

3. Verfahren nach den Ansprüchen 1 oder 2, dadurch gekennzeichnet, daß man die Reduktion in einem aprotischen Lösungsmittel vornimmt.

4. Verfahren nach den Ansprüchen 1 bis 3, dadurch gekennzeichnet, daß man als aprotisches Lösungsmittel Tetrahydrofuran verwendet.

5. Verfahren zur Herstellung von (3S)-3-Hydroxyoctandisäurediester II gemäß Anspruch 1, dadurch gekennzeichnet, daß man einen 3-Oxooctandisäurediester der allgemeinen Formel III

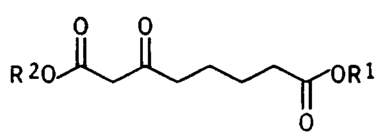

III

mit Bäckerhefe reduziert.

6. Verfahren nach Anspruch 5, dadurch gekennzeichnet, daß $R^1$ in der Verbindung III eine Methylgruppe und $R^2$ eine Isobutylgruppe bedeuten.

7. (3S)-3-Hydroxyoctandisäurediester der allgemeinen Formel II gemäß Anspruch 1.

8. (3S)-3-Hydroxyoctandisäurediester der allgemeinen Formel IIa

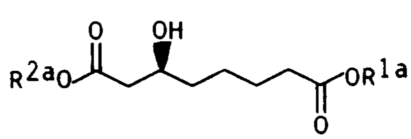

IIa

in der
$R^{1a}$ und $R^{2a}$ $C_1$-$C_{20}$-Alkylgruppen, $C_3$-$C_{12}$-Cycloalkylgruppen, $C_7$-$C_{12}$-Aralkylgruppen und/oder ein-

oder zweikernige Arylgruppen bezeichnen.

**9.** 3-Oxooctandisäurediester der allgemeinen Formel IIIb

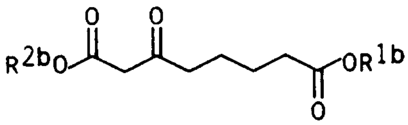

IIIb

in der
$R^{1b}$ und $R^{2b}$ $C_1$-$C_{20}$-Alkylgruppen, $C_3$-$C_{12}$-Cycloalkylgruppen, $C_7$-$C_{12}$-Aralkylgruppen und/oder ein- oder zwei kernige Arylgruppen bezeichnen, ausgenommen die Methylgruppe für $R^{1b}$ und die Methyl- oder tert.-Butylgruppe für $R^{2b}$ sowie die Ethylgruppe für $R^{1b}$ und $R^{2b}$.

**10.** Verfahren zur Herstellung von R(+)-α-Liponsäure der Formel IV

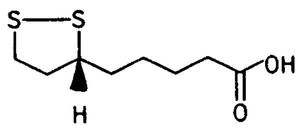

IV

dadurch gekennzeichnet, daß man die Verbindungen II gemäß Anspruch 1 zu den Verbindungen I reduziert und diese in an sich bekannter Weise
a) in organischer Lösung mit einem Sulfonsäurechlorid und einer tertiären Stickstoffbase in den Bissulfonsäureester von I überführt,
b) diese Verbindung in einem polaren Lösungsmittel mit Schwefel und einem Alkalimetalldisulfid zum R-α-Liponsäureester umsetzt und
c) diesen Ester gewünschtenfalls in die physiologisch verträgliche R-(+)-α-Liponsäure überführt.

**Patentansprüche für folgenden Vertragsstaat : ES**

**1.** Verfahren zur Herstellung von (6S)-6,8-Dihydroxyoctansäureestern der allgemeinen Formel I

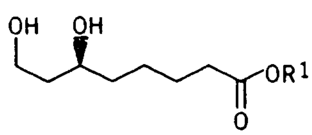

I

in der
$R^1$ eine Alkyl-, Cycloalkyl-, Aralkyl- oder Arylgruppe bezeichnet, dadurch gekennzeichnet, daß man einen (3S)-3-Hydroxyoctandisäurediester der allgemeinen Formel II

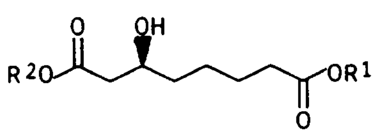

II

in der $R^2$ einen der Reste $R^1$ bedeutet, mit einem komplexen Hydrid reduziert.

**2.** Verfahren nach Anspruch 1, dadurch gekennzeichnet, daß man als komplexes Hydrid Natriumborhydrid verwendet.

**3.** Verfahren nach den Ansprüchen 1 oder 2, dadurch gekennzeichnet, daß man die Reduktion in einem aprotischen Lösungsmittel vornimmt.

**4.** Verfahren nach den Ansprüchen 1 bis 3, dadurch gekennzeichnet, daß man als aprotisches Lösungsmittel Tetrahydrofuran verwendet.

**5.** Verfahren zur Herstellung von (3S)-3-Hydroxyoctandisäurediester II gemäß Anspruch 1, dadurch gekennzeichnet, daß man einen 3-Oxooctandisäurediester der allgemeinen Formel III

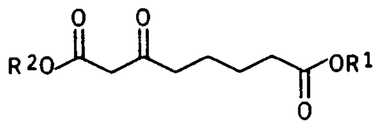

III

mit Bäckerhefe reduziert.

**6.** Verfahren nach Anspruch 5, dadurch gekennzeichnet, daß $R^1$ in der Verbindung III eine Methylgruppe und $R^2$ eine Isobutylgruppe bedeuten.

**7.** Verfahren zur Herstellung von R-(+)-α-Liponsäure der Formel IV

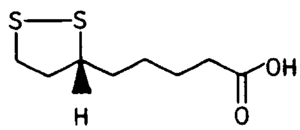

IV

dadurch gekennzeichnet, daß man die Verbindungen II gemäß Anspruch 1 zu den Verbindungen I reduziert und diese in an sich bekannter Weise

a) in organischer Lösung mit einem Sulfonsäurechlorid und einer tertiären Stickstoffbase in den Bissulfonsäureester von I überführt,

b) diese Verbindung in einem polaren Lösungsmittel mit Schwefel und einem Alkalimetalldisulfid zum R-α-Liponsäureester umsetzt und

c) diesen Ester gewünschtenfalls in die physiologisch verträgliche R-(+)-α-Liponsäure überführt.

**Claims**

**Claims for the following Contracting States : CH, DE, FR, GB, IT, LI, SE**

**1.** A process for preparing a (6S)-6,8-dihydroxyoctanoic ester of the formula I

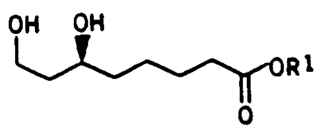

I

where

$R^1$ is alkyl, cycloalkyl, aralkyl or aryl, which comprises reducing a (3S)-3-hydroxyoctanedioic diester of the formula II

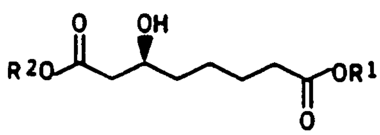

II

where $R^2$ is one of the $R^1$ radicals, with a complex hydride.

**2.** A process as claimed in claim 1, wherein sodium borohydride is used as complex hydride.

**3.** A process as claimed in claims 1 or 2, wherein the reduction is carried out in an aprotic solvent.

**4.** A process as claimed in claims 1 to 3, wherein tetrahydrofuran is used as aprotic solvent.

**5.** A process for preparing a (3S)-3-hydroxyoctanedioic diester II as defined in claim 1, which comprises reducing a 3-oxooctanedioic diester of the formula III

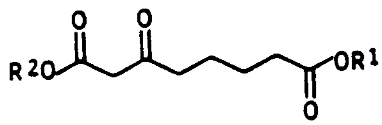

III

with baker's yeast.

**6.** A process as claimed in claim 5, wherein $R^1$ in the compound III is methyl and $R^2$ is isobutyl.

**7.** A (3S)-3-hydroxyoctanedioic diester of the formula II in claim 1.

**8.** A (3S)-3-hydroxyoctanedioic diester of the formula IIa

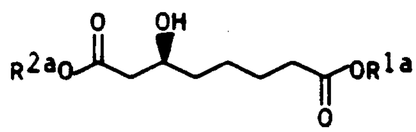

IIa

where $R^{1a}$ and $R^{2a}$ are each $C_1$-$C_{20}$-alkyl, $C_3$-$C_{12}$-cycloalkyl, $C_7$-$C_{12}$-aralkyl or mononuclear or dinuclear aryl.

**9.** A 3-oxooctanedioic diester of the formula IIIb

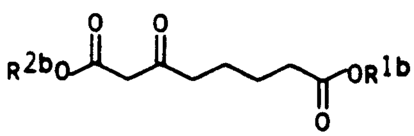

IIIb

where $R^{1b}$ and $R^{2b}$ are each $C_1$-$C_{20}$-alkyl, $C_3$-$C_{12}$-cycloalkyl, $C_7$-$C_{12}$-aralkyl or mononuclear or binuclear aryl, excepting methyl for $R^{1b}$ and methyl or tert-butyl for $R^{2b}$, and ethyl for $R^{1b}$ and $R^{2b}$.

**10.** A process for preparing R-(+)-α-lipoic acid of the formula IV

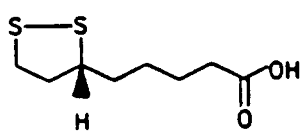

IV

which comprises reducing a compound II as defined in claim 1 to a compound I and, in a conventional manner,

a) converting the latter in organic solution with a sulfonyl chloride and a tertiary nitrogen base into the bissulfonic ester of I,

b) reacting this compound in a polar solvent with sulfur and an alkali metal disulfide to give an R-α-lipoic ester and

c) if required converting this ester into physiologically tolerated R-(+)-α-lipoic acid.

**Claims for the following Contracting State : ES**

**1.** A process for preparing a (6S)-6,8-dihydroxyoctanoic ester of the formula I

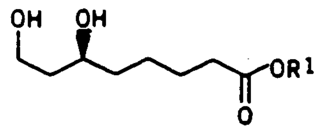

I

where

$R^1$ is alkyl, cycloalkyl, aralkyl or aryl, which comprises reducing a (3S)-3-hydroxyoctanedioic diester of the formula II

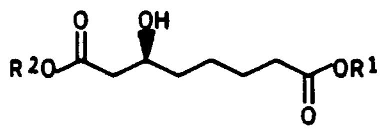

II

where $R^2$ is one of the $R^1$ radicals, with a complex hydride.

**2.** A process as claimed in claim 1, wherein sodium borohydride is used as complex hydride.

**3.** A process as claimed in claims 1 or 2, wherein the reduction is carried out in an aprotic solvent.

**4.** A process as claimed in claims 1 to 3, wherein tetrahydrofuran is used as aprotic solvent.

**5.** A process for preparing a (3S)-3-hydroxyoctanedioic diester II as defined in claim 1, which comprises reducing a 3-oxooctanedioic diester of the formula III

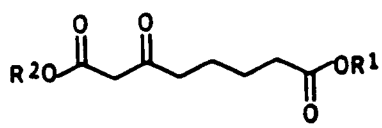

III

with baker's yeast.

**6.** A process as claimed in claim 5, wherein $R^1$ in the compound III is methyl and $R^2$ is isobutyl.

**7.** A process for preparing R-(+)-α-lipoic acid of the formula IV

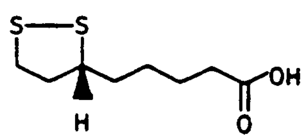

IV

which comprises reducing a compound II as defined in claim 1 to a compound I and, in a conventional manner,

a) converting the latter in organic solution with a sulfonyl chloride and a tertiary nitrogen base into the bissulfonic ester of I,

b) reacting this compound in a polar solvent with sulfur and an alkali metal disulfide to give an R-α-lipoic ester and

c) if required converting this ester into physiologically tolerated R-(+)-α-lipoic acid.

**Revendications**

**Revendications pour les Etats contractants suivants : CH, DE, FR, GB, IT, LI, SE**

1. Procédé de préparation d'esters d'acide (6S)-6,8-dihydroxyoctanoïque de formule générale I

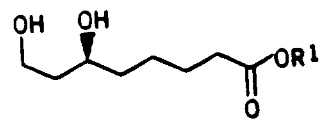

I

dans laquelle
$R^1$ représente un groupement alkyle, cycloalkyle, aralkyle ou aryle, caractérisé an ce que l'on réduit avec un hydrure complexe un diester d'acide (3S)-3-hydroxyoctanedioïque de formule générale II

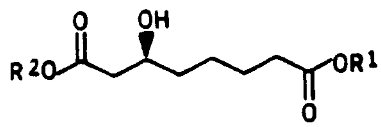

II

dans laquelle $R^2$ représente l'un des restes $R^1$.

2. Procédé selon la revendication 1, caractérisé en ce que l'on utilise, comme hydrure complexe, du borohydrure de sodium.

3. Procédé selon la revendication 1 ou 2, caractérisé an ce que l'on mène la réduction dans un solvant aprotique.

4. Procédé selon l'une quelconque des revendications 1 à 3, caractérisé an ce que l'on utilise du tétrahydrofuranne comme solvant aprotique.

5. Procédé de préparation de diesters d'acide (3S)-3-hydroxyoctanedioïque II selon la revendication 1, caractérisé en ce que l'on réduit avec de la levure de boulanger un diester d'acide 3-oxooctanedioïque de formule générale III

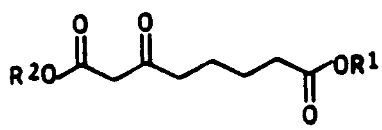

III

6. Procédé selon la revendication 5, caractérisé en ce que $R^1$ dans le composé III représente un groupement méthyle et $R^2$ représente un groupement isobutyle.

7. Diesters d'acide (3S)-3-hydroxyoctanedioïque de formule générale II selon la revendication 1.

8. Diesters d'acide (3S)-3-hydroxyoctanedioïque de formule générale IIa

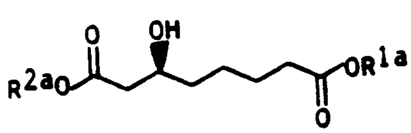

IIa

dans laquelle
$R^{1a}$ et $R^{2a}$ représentent des groupements alkyle en $C_1$-$C_{20}$, des groupements cycloalkyle en $C_3$-$C_{12}$,

des groupements aralkyle en $C_7$-$C_{12}$ et/ou des groupements aryle à un ou deux noyaux.

**9.** Diesters d'acide 3-oxooctanedioïque de formule générale IIIb

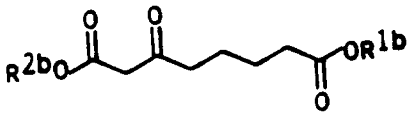

IIIb

dans laquelle
$R^{1b}$ et $R^{2b}$ représentent des groupements alkyle en $C_1$-$C_{20}$, des groupements cycloalkyle en $C_3$-$C_{12}$, des groupements aralkyle en $C_7$-$C_{12}$ et/ou des groupements aryle à un ou deux noyaux, à l'exception du groupement méthyle pour $R^{1b}$ et du groupement méthyle ou tert.-butyle pour $R^{2b}$, ainsi que du groupement éthyle pour $R^{1b}$ et $R^{2b}$.

**10.** Procédé de préparation d'acide R-(+)-α-lipoïque de formule IV

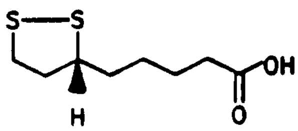

IV

caractérisé en ce que l'on réduit les composés II selon la revendication 1 en les composés I et, de façon connue en soi,

a) on transforme ceux-ci, en solution organique, en l'ester d'acide bissulfonique de I avec un chlorure de sulfonyle et une base azotée tertiaire,

b) on fait réagir ce composé, dans un solvant polaire, avec du soufre et un disulfure de métal alcalin pour obtenir l'ester d'acide R-α-lipoïque et

c) on transforme éventuellement cet ester en l'acide R-(+)-α-lipoïque acceptable physiologiquement.

**Revendications pour l'Etat contractant suivant : ES**

**1.** Procédé de préparation d'esters d'acide (6S)-6,8-dihydroxyoctanoïque de formule générale I

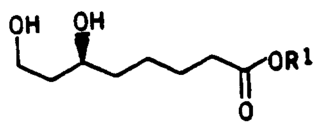

I

dans laquelle
$R^1$ représente un groupement alkyle, cycloalkyle, aralkyle ou aryle, caractérisé en ce que l'on réduit avec un hydrure complexe un diester d'acide (3S)-3-hydroxyoctanedioïque de formule générale II

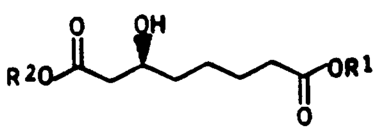

II

dans laquelle $R^2$ représente l'un des restes $R^1$.

**2.** Procédé selon la revendication 1, caractérisé en ce que l'on utilise, comme hydrure complexe, du borohydrure de sodium.

**3.** Procédé selon la revendication 1 ou 2, caractérisé en ce que l'on mène lu réduction dans un solvant aprotique.

**4.** Procédé selon l'une quelconque des revendications 1 à 3, caractérisé en ce que l'on utilise du tétrahydrofuranne comme solvant aprotique.

**5.** Procédé de préparation de diesters d'acide (3S)-3-hydroxyoctanedioïque II selon la revendication 1, caractérisé en ce que l'on réduit avec de la levure de boulanger un diester d'acide 3-oxooctanedioïque de formule générale III

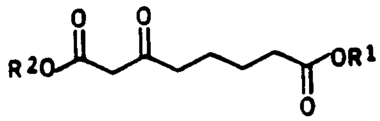

III

**6.** Procédé selon la revendication 5, caractérisé en ce que $R^1$ dans le composé III représente un groupement méthyle et $R^2$ représente un groupement isobutyle.

**7.** Procédé de préparation d'acide R-(+)-α-lipoïque de formule IV

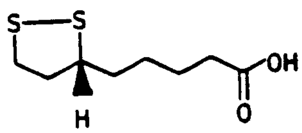

IV

caractérisé en ce que l'on réduit les composés II selon la revendication 1 en les composés I et, de façon connue en soi,

a) on transforme ceux-ci, en solution organique, en l'ester d'acide bissulfonique de I avec un chlorure de sulfonyle et une base azotée tertiaire,

b) on fait réagir ce composé, dans un solvant polaire, avec du soufre et un disulfure de métal alcalin pour obtenir l'ester d'acide R-α-lipoïque et

c) on transforme éventuellement cet ester en l'acide R-(+)-α-lipoïque acceptable physiologiquement.